# EUROPÄISCHE PATENTANMELDUNG

(11) **EP 1 092 701 A1**
(43) Veröffentlichungstag der Anmeldung: **18.04.2001**
(21) Anmeldenummer: 00116668.5
(22) Anmeldetag: 02.08.2000
(51) Int. Cl.: C07C 46/08, C07C 50/02

(54) **Verfahren zur Herstellung von 2,3,5-Trimethyl-p-benzochinon**

(30) Priorität: 15.10.1999 DE 19949795
(71) Anmelder: Degussa-Hüls Aktiengesellschaft, 60287 Frankfurt am Main (DE)
(72) Erfinder: Maassen, Ralf, Dr., 63457 Hanau (DE); Krill, Steffen, Dr., 67346 Speyer (DE); Jäger, Barbara, 63579 Freigericht (DE); Huthmacher, Klaus, Dr., 63584 Gelnhausen (DE)

(57) **Zusammenfassung**

Verfahren zur Herstellung von 2,3,5-Trimethyl-p-benzochinon durch Oxidation von 2,3,5-Trimethylphenol oder 2,3,6-Trimethylphenol mit Sauerstoff oder einem Sauerstoffenthaltendem Gasgemisch in Gegenwart eines Katalysatorsystems bestehend aus Kupferhalogenid und einem Übergangsmetallhalogenid aus der Gruppe Eisen, Chrom, Mangan, Kobalt, Nickel, Zink oder einem Halogenid der seltenen Erden in einem zweiphasigen Reaktionsmedium bei erhöhter Temperatur.

## Beschreibung

Die vorliegende Erfindung betrifft ein neues Verfahren zur Herstellung von 2,3,5-Trimethyl-p-benzochinon durch Oxidation von Phenolen mittels Sauerstoff in Gegenwart eines zweiphasigen flüssigen Reaktionsmediums mit einem Katalysatorgemisch aus Kupferchlorid und zusätzlich einem Übergangsmetallhalogenid aus der Gruppe Eisen, Chrom, Mangan, Kobalt, Nickel oder Zink oder einem Halogenid eines Elements der seltenen Erden. Als Reaktanden können hierbei sowohl 2,3,5-Trimethylphenol als auch 2,3,6-Trimethylphenol eingesetzt werden.

2,3,5-Trimethyl-p-benzochinon ist ein Zwischenprodukt, das unter anderem für die Herstellung von α-Tocopherolen (Vitamin E)verwendet wird.

Die Oxidation von Trimethylphenolen zu 2,3,5-Trimethyl-p-benzochinon ist bekannt.

Die Verwendung von anorganischen Oxidationsmitteln, inklusive Kaliumpermanganat, Mangandioxid und Bleioxid ist beschrieben worden, wobei in vorbekannten Verfahren der Einsatz stöchiometrischer Mengen des Oxidationsmittels notwendig ist. Die Verwendung stöchiometrischer Mengen dieser teuren Oxidationsmittel verursacht einen hohen Chemikalienverbrauch und erzeugt Abfallströme, die mit den entsprechenden reduzierten Metallen belastet sind und die unter erhöhtem Aufwand regeneriert oder entsorgt werden müssen.

Weiterhin sind katalytische Verfahren bekannt, die die Trimethylphenol Oxidation in Gegenwart eines Metallkatalysators mit einem sauerstoffenthaltenden Gas als Oxidationsmittel durchführen. Eine technische Umsetzung dieser Verfahren, beispielsweise unter Verwendung eines Kobalt-Salen-Komplexkatalysators, ist aufgrund der geringen Katalysatorlebensdauer aufwendig und teuer, da es notwendig ist, nicht unerhebliche Mengen an frischem Katalysator nachzudosieren und erhebliche Mengen an mit Metallen belastetem Ausschleusstrom zu entsorgen oder aufwendig aufzuarbeiten.

Beispielsweise wird in der EP 0 659 727 als Sauerstoff übertragender Katalysator Tetraaza [14] annulen beschrieben, der ein Komplex gebundenes Schwermetallion enthält. Dieser Katalysatorkomplex wird bei der Oxidation zerstört und ist nicht recyclierbar, so daß er sich nicht für den technischen Einsatz eignet.

In diesem Zusammenhang ist in der US 3796 732 die Verwendung von Kupferchlorid als Katalysator der Umsetzung beschrieben, wobei in homogener Phase in Gegenwart eines inerten Lösungsmittels wie DMF gearbeitet wird und sich das technisch nur aufwendig zu lösende Problem der Katalysatoraufarbeitung ergibt.

Eine Verbesserung der Ausbeuten wird im JP 17585/1978 unter Verwendung eines Katalysatorsystems bestehend aus Kupferionen und Halogenionen beschrieben. Nachteilig an diesem Verfahren sind trotz guter Ausbeuten die niedrige Raum-Zeit Ausbeute und die Notwendigkeit, den Katalysator mit großen Wassermengen zu extrahieren, Wasser zur Recyclierung des Katalysators zu entfernen und nicht zuletzt der negative Einfluß von Restwasser auf die Katalysatorleistung des recyclierten Katalysators.

In der JP 93931/1975 werden zur Erhaltung der Katalysatoraktivität bei der Recyclierung Halogene oder halogenierte Verbindungen zugesetzt, die sich jedoch unter den Reaktionsbedingungen schnell verbrauchen und daher regelmäßig ergänzt werden müssen. Dies ist verfahrenstechnisch aufwendig und führt zu deutlich erhöhten Produktionskosten.

Eine Möglichkeit, die Probleme der Katalysatorrecyclierung unter gleichzeitigem Erhalt der Katalysatoraktivität zu umgehen, wird im RU-2 039 037 beschrieben, in dem die Oxidation von Trimethylphenol und strukturverwandten Verbindungen in Gegenwart eines heterogenen Katalysators mittels Sauerstoff oder eines sauerstoffenthaltenden Gases offengelegt wird.

Als nachteilig an diesem Verfahren erweist sich die aufwendige Präparation des heterogenen Katalysators, der durch Aufbringen eines einwertigen Kupferchlorids in Gegenwart von Ammoniumchlorid und Alkalimetallchlorid auf Aluminiumhydroxid als Träger in Gegenwart einer definierten Menge Phosphorsäure erhalten wird.

Nach EP 0 127 888 werden wäßrige Lösungen von Li(CuCl₃)in Gegenwart eines hohen Überschusses an dem entsprechenden Lithiumhalogenid als Oxidationskatalysator eingesetzt. Es zeigt sich jedoch, daß trotz guter Ausbeuten eine technische Umsetzung dieses Verfahrens nicht vorteilhaft ist, weil große Überschüsse an teurem Lithiumhalogenid eingesetzt werden müssen, der komplexe Kupfer-(II)-Katalysator vor der Reaktion aufwendig hergestellt und zum Erzielen guter Ausbeuten mindestens äquivalente Mengen des Katalysators bezogen auf Trimethylphenol eingesetzt werden müssen.

In der EP 0 167 153 ist die Verwendung einer wäßrigen Katalysatorlösung bestehend aus Li(CuCl₃)oder entsprechender Kupfer-(II)-Komplexe in Gegenwart eines Überschusses an dem entsprechenden Lithiumhalogenid beschrieben.

Auch in der EP 0 294 584 wird ein Verfahren zur Herstellung von 2,3,5-Trimethyl-p-benzochinon in Gegenwart eines Katalysators bestehend aus Kupfer-(II)-chlorid und Lithiumchlorid in einem zweiphasigen Reaktionsmedium, bestehend aus Wasser und einer Mischung aus einem aromatischen Kohlenwasserstoff und einem niedrigen aliphatischen Alkohol mit 1-4 C-Atomen beschrieben. Die Verwendung eines komplexen organischen Lösungsmittelgemisches, das nach der Reaktion destillativ aufgearbeitet werden muß, ist aus technischer Sicht nicht vorteilhaft.

Eine andere Variante der Oxidation in einem zweiphasigen Reaktionssystem wird in EP 0 369 824 beschrieben. Der Katalysator besteht aus einem binären System bestehend aus einem Kupfer-(II)-halogenid und einer stickstoffhaltigen Verbindung, vorzugsweise einem Hydroxylamin, einem Oxim oder Amin oder den entsprechenden Ammoniumsalzen. Als nachteilig erweist sich, daß die stickstoffhaltige Katalysatorkomponente unter oxidativen Bedingungen abgebaut wird, nicht recycliert werden kann und daher hohe Kosten verursacht.

In der EP 0 475 272 wird die Oxidation in Gegenwart eines sauerstoffhaltigen Gases unter Verwendung eines Katalysators bestehend aus Kupfer-(II)-halogenid und einem Erdalkalihalogenid in einem zweiphasigen Lösungsmittelystem bestehend aus Wasser und einem gesättigten aliphatischen Alkohol mit 5-10 C-Atomen beschrieben. In diesem Verfahren wird der aktive Katalysator in situ aus den Kupfer-(II)-salzen und den Erdalkaliadditiven gebildet und das organische Lösungsmittelsystem besitzt einen im Vergleich zu den angewendeten Reaktionstemperaturen ausreichend hohen Flammpunkt. Jedoch muß hier zum Erreichen guter Umsätze und Ausbeuten der Katalysator in stöchiometrischen Mengen zugesetzt werden.

Die in EP 0 387 820 beschriebende Reaktionsführung in aliphatischen Alkoholen mit 12 bis 18 C-Atomen gestattet ebenfalls die Oxidation bei Temperaturen unterhalb des Flammpunktes des organischen Lösungsmittels, allerdings ist das Verfahren technisch wenig attraktiv, da sich die Reaktionsführung und die Isolierung des 2,3,5-Trimethyl-p-benzochinons aufgrund der relativ hohen Schmelz- und Siedepunkte der Alkohole sehr aufwendig gestalten.

Aufgabe der vorliegenden Erfindung ist es, ein neues Verfahren zur Herstellung von 2,3,5 Trimethyl-p-benzochinon bereitzustellen, um die im Stand der Technik beschriebenen, zum Teil erheblichen Nachteile bezüglich der Einsatzstoffkosten, des Aufarbeitungsaufwandes und nicht zuletzt bezüglich sicherheitstechnischer Aspekte, die die Umsetzung im technischen Maßstab verhindern, zu lösen. Es war insbesondere Aufgabe der vorliegenden Erfindung, die folgenden Anforderungen an den Prozeß zu erfüllen:
a.) Verwendung eines Katalysatorsystems bestehend aus günstigen, am Markt frei verfügbaren Einsatzstoffen, die die aktive Katalysatorspezies in situ unter den gegebenen Reaktionsbedingungen generieren, im Gegensatz zu den bislang beschriebenen Katalysatoren, die teilweise in separaten Verfahrensschritten vor der eigentlichen Oxidationsreaktion hergestellt werden müssen oder sich während der Reaktion verbrauchen.
b.) Verwendung eines Katalysatorsystems hoher Aktivität und gleichzeitig großer Lebensdauer, das nach der Reaktion ohne besondere Maßnahmen recycliert und wiederholt eingesetzt werden kann.
c.) Verwendung eines Reaktionssystems bestehend aus verschiedenen, bei Raumtemperatur nicht mischbaren Phasen wobei die eine Phase den Katalysator in gelöster oder suspendierter Form enthält und eine weitere Phase das Substrat und während der Reaktion gebildetes Produkt in gelöster Form enthält, was eine sich der Reaktion anschließende Trennung von Substrat/Produktphase einerseits und Katalysatorphase andererseits ermöglicht und damit eine einfache Produktisolierung in hoher Ausbeute erlaubt und eine Recyclierung der Katalysatorphase unter geringem Aufwand ermöglicht.

Die Lösung dieser Aufgabe wird dadurch erreicht, daß Trimethylphenol durch Oxidation mit Sauerstoff oder einem Sauerstoff enthaltenden Gasgemisch in Gegenwart eines mindestens Kupferhalogenid enthaltenden Katalysators in einem zweiphasigen Reaktionsmedium bei erhöhter Temperatur umgesetzt wird. Das Verfahren ist dadurch gekennzeichnet, daß man die Umsetzung im Reaktionsmedium aus Wasser und einem aliphatischen Alkohol mit 5 bis 10 C-Atomen oder aus Wasser und einem aliphatischen Alkohol mit 1 bis 4 C-Atomen und einem aromatischen Kohlenwasserstoff in Gegenwart eines aus einem Kupferhalogenid und zusätzlich einem Übergangsmetallhalogenid der Gruppe Eisen, Chrom, Mangan, Kobalt, Nickel, Zink oder einem Halogenid eines Elementes der seltenen Erden bestehenden Katalysatorsystems bei Temperaturen zwischen 20 und 120°C durchführt.

Die Umsetzung kann in der Weise durchgeführt werden, daß die das Substrat Trimethylphenol enthaltende organische Phase aus einem geeigneten, bei Raumtemperatur nicht oder nur geringfügig in Wasser löslichen Lösungsmittel mit der wäßrigen, das Katalysatorsystem enthaltenden Phase in Kontakt gebracht wird, und die so hergestellte Reaktionsmischung mit einem sauerstoffenthaltenden Gas in Kontakt gebracht wird und nach Reaktionsende die organische Produktphase von der wäßrigen, noch aktiven Katalysatorphase abgetrennt wird, um das Produkt 2,3,5 Trimethyl-p-benzochinon zu isolieren.

Dieses Ergebnis war nicht zu erwarten, da in wäßrigen Systemen von Kupferhalogeniden und Übergangsmetallhalogeniden bzw. Halogeniden der Elemente der seltenden Erden mit der Bildung von schwerlöslichen, teilweise oligomeren oder polymeren Hydrolyseprodukten gerechnet werden muß, die keine selektive katalytische Wirkung für die untersuchte Oxidation zeigen.

Es zeigt sich bei der vorliegenden Erfindung, daß bei Verwendung eines binären Katalysatorsystems bestehend aus Kupferhalogeniden einerseits und Übergangsmetallhalogeniden oder Halogeniden von Elementen aus der Gruppe der seltenen Erden andererseits auch bei wiederholtem Einsatz der wäßrigen Katalysatorphase keine oder nur eine unwesentliche Katalysatordesaktivierung eintritt, und die Oxidation zu 2,3,5-Trimethyl-p-benzochinon unter den neuen Bedingungen in sowohl ökonomischer wie technisch vorteilhafter Weise durchgeführt werden kann.

Es lassen sich auch bei wiederholtem Einsatz der Katalysatorphase Aubeuten an 2,3,5-Trimethyl-p-benzochinon von über 90% erzielen. Die Verwendung ausgewählter Übergangsmetallhalogenide wie beispielsweise CrCl₃, FeCl₃ oder ZnCl₂ bietet einen weiteren wirtschaftlichen Vorteil gegenüber etwa der Verwendung des teuren LiCl.

Als Oxidationsmittel im erfindungsgemäßen Verfahren dient Sauerstoff in reiner Form oder in verdünnter Form z. B. Luft. Bezogen auf 1 L Reaktionsgemisch werden dabei in der Regel pro Stunde 10 bis 150 NL gasförmiger Sauerstoff zugeführt. Als im Sinne der Erfindung geeignete Kupfersalze seien, ohne Anspruch auf Vollständigkeit, im wesentlichen CuCl₂ und CuBr₂, oder entsprechende Cu-(I)-salze wie CuCl oder CuBr, insbesondere CuCl₂ und CuCl genannt. Bevorzugt wird hierbei Cu(II)chlorid eingesetzt.

Als im Sinne der Erfindung geeignete Übergangsmetallhalogenide seien im wesentlichen Chloride der Übergangsmetalle genannt. Besonders geeignet sind die Elementenhalogenide der vierten Periode, wie zum Beispiel Halogenide der Elemente Cr, Mn, Fe, Co, Ni und Zn, sowie Ce aus der Gruppe der seltenen Erden.

Als Reaktionsmedium eignen sich im Gemisch mit Wasser insbesondere verzweigte und unverzweigte aliphatische C₅-C₁₀ Alkohole wie 1-Pentanol, 1-Hexanol, 1-Heptanol, 1-Octanol, 1-Nonanol, 1-Decanol, 2-Ethylhexanol oder Cyclohexanol.

Als Reaktionsmedium eignen sich ebenso im Gemisch mit Wasser und einem aromatischen Kohlenwasserstoff die verzweigten und unverzweigten aliphatischen Alkohole wie Methanol, Ethanol, n-Propanol, Isopropanol, 1-Butanol, 2-Butanol undtert. Butanol.

Als aromatische Kohlenwasserstoffe werden vorzugsweise solche mit 6 bis 8 Kohlenstoffatomen verwendet, insbesondere Benzol, Toluol, Xylole oder halogensubstituierte Aromaten wie Chlorbenzol.

Die Darstellung der wäßrigen Katalysatorphase erfolgt durch einfache Mischung der wäßrigen Lösungen der Einzelkomponenten oder durch Auflösen der festen Salzverbindungen in Wasser, was die Prozeßführung deutlich vereinfacht.

Das molare Verhältnis des Kupferhalogenids bezüglich Trimethylphenol ist in weiten Bereichen variierbar und beträgt üblicherweise Kupfersalz/Trimethylphenol = 0,1 - 10, bevorzugt 0,2 - 3.

Die Übergangsmetallhalogenide können bezogen auf Trimethylphenol in der 0,1 bis 10fachen Menge eingesetzt werden, bevorzugt wird eine 0,2 bis 5fache molare Menge. Die Konzentration des Kupferhalogenids in der wäßrigen Katalysatorphase kann im Falle der Verwendung der Kupfer-(II)-salze zwischen 1-70 Gew.-% variiert werden, vorzugsweise werden Konzentrationen zwischen 5-30 Gew.-% verwendet, die Übergangsmetallhalogenide bzw. die Halogenide der seltenen Erden werden bevorzugt in einem Konzentrationsbereich zwischen 5 - 80 Gew.-% verwendet.

Als zusätzliche Aktivatoren der Umsetzungen können die aus dem Stand der Technik bekannten Systeme eingesetzt werden, am vorteilhaftesten werden Kupfersalze wie Kupfer-(I)-chlorid oder das entsprechende Hydroxid eingesetzt.

Zweiphasige Gemische, die z. B. aus der Anwendung von Wasser und einem nicht oder nur begrenzt mit Wasser mischbaren Lösungsmittel resultieren, werden gegebenenfalls mit einem Phasentransferkatalysator versehen. Als Phasentransferkatalysatoren kommen dabei die üblichen, an sich bekannten Produkte, wie Tetraalkylammoniumhalogenide, Benzyltrialkylammoniumhalogenide oder -hydrogensulfate sowie die entsprechenden Phosphoniumsalze und auch Verbindungen aus der Reihe der Polyethylenglykole in Betracht. Das neue Verfahren wird in der Regel bei Normaldruck und einer Temperatur von 20 bis 120°C durchgeführt. Das Verfahren kann ebenfalls unter Druck durchgeführt werden; eine Druckfahrweise ist insbesondere bei sauerstoffhaltigen Gasgemischen angebracht. Es kann sowohl in kontinuierlicher als auch diskontinuierlicher Arbeitsweise vorgenommen werden.

Zur Durchführung der Reaktion wird Trimethylphenol in der organischen Komponente des Lösungsmittelsystems gelöst und zur wäßrigen, den Katalysator enthaltenden Phase zudosiert. In einer anderen Ausführungsform wird ein Teil des organischen Lösungsmittels vor Reaktionsbeginn mit der wäßrigen Phase vorgelegt und die Trimethylphenol-Lösung zudosiert. In wiederum einer anderen Variante der Reaktionsführung wird die Reaktion batchweise durchgeführt, indem alle Komponenten unter Rühren vorgelegt werden und anschließend mit der Zudosierung des sauerstoffhaltigen Gases begonnen wird.

Die Konzentration von Trimethylphenol in der organischen Phase kann in weiten Konzentrationsbereichen variiert werden, im allgemeinen werden Trimethylphenol-Konzentrationen zwischen 5 und 80% eingestellt, bevorzugt Konzentrationen zwischen 10 und 50%.

Das Volumenverhältnis von Wasser zu organischem Lösungsmittel kann in einem Bereich zwischen 10:1 und 1:10 schwanken, bevorzugt wird ein Bereich zwischen 3:1 und 1:5.

Die Reaktionstemperatur kann über ein weites Temperaturintervall variiert werden, bevorzugt wird die Umsetzung zwischen 20 und 120°C durchgeführt, in einer besonders bevorzugten Ausführungsform wird zwischen 40 und 90°C gearbeitet.

Das Reaktionsprodukt 2,3,5-Trimethyl-p-benzochinon kann auf üblichem Weg, z. B. mittels Vakuum- und Wasserdampfdestillation isoliert werden.

Das erfindungsgemäße Verfahren ist einfach durchzuführen und liefert das Reaktionsprodukt in guter Ausbeute und hoher Reinheit.

Die Ausbeutebestimmungen wurden auf einem HP 5890 oder einem HP 6890 Gaschromatographen unter Verwendung einer J&W DB-5 Kapillarsäule mit 30 m Länge, 0,32 mm Innendurchmesser und 1µm Filmdicke durchgeführt. Als interner Standard diente Tetradecan. Als Referenzsubstanz diente TMQ, welches durch Destillation und mehrfache Kristallisation gereinigt wurde.

Die HPLC-Messungen wurden auf einem System der Firma Jasco durchgeführt, bestehend aus einem UV-Detektor UV 975, einer Pumpe PU 980 und einem Autosampler AS 950. Die verwendete Säule war eine Intersil-ODS 3V-5µ, 250 x 4,6 mm Innendurchmesser der Firma GL Sciences Inc.. Als externer Standard diente die oben beschriebene TMQ-Referenzsubstanz.

Die folgenden Beispiele sollen die Erfindung näher erläutern.
TMP steht für Trimethylphenol
TMQ steht für 2,3,5 Trimethyl-p-benzochinon.

### Beispiele

### Beispiel 1:

In einem 100 ml Dreihalskolben wurden 2,98 g FeCl₃ (18,4 mmol) und 0,91 g (9,2 mmol) CuCl in Wasser gelöst (molares Verhältnis CuCl : FeCl₃ = 0,5). Die Katalysatorkonzentration des binären Salzgemisches berug 13,5 Gew.-% in der wäßrigen Phase. Zu dieser wäßrigen Katalysatorphase wurde unter lebhaftem Rühren eine Lösung von 2,5 g TMP (=18,4 mmol) in 25 ml Hexanol gegeben. Die TMP Konzentration in der organischen Phase betrug 11 Gew.-%. Unter Sauerstoffbegasung über eine Fritte wurde die Reaktionsmischung auf 60°C aufgeheizt und der Reaktionsverlauf mittels Gaschromatographie verfolgt. Nach Ablauf der Reaktion wurde eine TMQ-Ausbeute von 82,2% erhalten.

### Beispiel 2 bis 6:

Analog Beispiel 1 wurden in einem 100 ml Dreihalskolben die Komponenten vorgelegt, wobei das Verhältnis TMP : CuCl₂ : FeCl₃=l : 0,75 : 1,5 betrug. Die Konzentration des binären Katalysators in der Wasserphase betrug bei allen Versuchen 39,4 Gew.-%. Zu der vorgelegten Katalysatorphase wurde die TMP-Alkohol-Lösung hinzugegeben, anschließend die Mischung auf die angegebene Temperatur gebracht und mit der Begasung mit Sauerstoff begonnen. Unter Variation der Reaktionstemperaturen und der Reaktionszeiten wurden nach Reaktionsende die folgenden Ergebnisse erhalten.

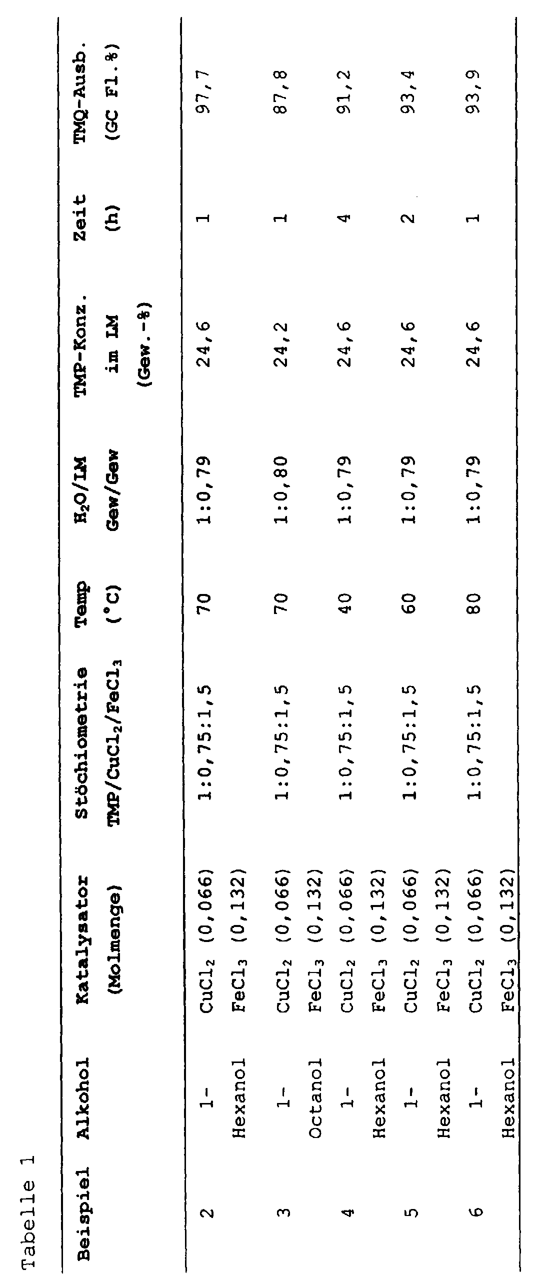

### Beispiel 7 bis 12

In einem Glasreaktor wurden Kupfer(II)chlorid und ein Übergangsmetallchlorid oder ein Chlorid eines Elements der seltenen Erden in den aus Tabelle 2 ersichtlichen Mengen als wäßrige Lösung vorgelegt, mit 40 mL 1-Hexanol versetzt und auf 65°C aufgeheizt. Anschließend wurde unter Rühren (900 Upm) und Sauerstoffbegasung über eine Fritte innerhalb von 3 h eine Lösung aus 12 g 2,3,6-Trimethylphenol (88 mmol) in 20mL 1-Hexanol zugetropft. Nach Beendigung der Zugabe wurde noch weitere 2 h bei 80°C unter Sauerstoffbegasung nachgerührt und der Reaktionsverlauf per HPLC verfolgt. Nach Ablauf der Reaktion wurden die Phasen getrennt, die organische Phase 2mal mit Wasser gewaschen und die TMQ-Ausbeute durch Gaschromatographie mit internem Standard ermittelt.

**Tabelle 2**

| | Katalysator (Molmenge [mmol]) | Katalysatorkonz. in wäßriger Phase (Gew.-%) | Stöchiometrie TMP/CuCl₂/Add. | H₂O /LM Gew./Gew. | TMQ-Ausbeute (%) |
|---|---|---|---|---|---|
| Beispiel 7 | CuCl₂ (0,066) | 39,0 | 1:0,75:1,5 | 1:1,05 | 94,3 |
| | CrCl₃ (0,132) | | | | |
| Beispiel 8 | CuCl₂ (0,066) | 40,9 | 1:0,75:1,5 | 1:1,33 | 93,7 |
| | MnCl₂ (0,132) | | | | |
| Beispiel 9 | CuCl₂ (0,066) | 35,8 | 1:0,75:1,5 | 1:1,05 | 91,1 |
| | CoCl₂ (0,132) | | | | |
| Beispiel 10 | CuCl₂ (0,066) | 38,4 | 1:0,75:1,5 | 1:1,18 | 93,0 |
| | NiCl₂ (0,132) | | | | |
| Beispiel 11 | CuCl₂ (0,066) | 50,7 | 1:0,75:0,75 | 1:2,82 | 89,9 |
| | ZnCl₂ (0,066) | | | | |
| Beispiel 12 | CuCl₂ (0,066) | 45,8 | 1:0,75:1,5 | 1:1,00 | 91,5 |
| | CeCl₃ (0,132) | | | | |

### Beispiel 13

In einem Glasreaktor wurden Kupfer(II)chlorid (66 mmol) und Chrom(III)chlorid (132 mmol) als wäßrige Lösung vorgelegt (Katalysatorkonzentration in der wäßrigen Phase: 39,0 Gew.-%), mit 40 ml 1-Hexanol versetzt und auf 65°C aufgeheizt. Anschließend wurde unter Rühren (900 Upm) und Sauerstoffbegasung über eine Fritte innerhalb von 3 h eine Lösung aus 12 g 2,3 6-Trimethylphenol (88mmol) in 20 mL 1-Hexanol zugetropft. Nach Beendigung der Zugabe wurde noch weitere 2 h bei 80 °C unter Sauerstoffbegasung nachgerührt und der Reaktionsverlauf per HPLC verfolgt. Nach Ablauf der Reaktion wurden die Phasen getrennt, die organische Phase 2mal mit Wasser gewaschen und die TMQ-Ausbeute durch Gaschromatographie mit internem Standard ermittelt. Die vereinigten wäßrigen Phasen wurden am Rotationsverdampfer auf das ursprüngliche Volumen eingeengt und wieder als Katalysatorlösung in den Glasreaktor überführt. Dieses Verfahren wurde mehrfach wiederholt.

**Tabelle 3**

| Beispiel 13, Lauf | Anzahl der Verfahrenswiederholungen | TMQ-Ausbeute (%) |
|---|---|---|
| 1 | 1 | 92,0 |
| 2 | 2 | 94,2 |
| 3 | 3 | 94,3 |
| 4 | 4 | 93,8* |
| 5 | 5 | 93,7 |
| 6 | 6 | 92,3 |
| 7 | 7 | 94,3 |

| | | |
|---|---|---|
| *HPLC-Analytik mit externem Standard | | |

### Beispiel 14 bis 17

In einem Glasreaktor wurden Kupfer(II)chlorid (66 mmol) und Chrom(III)chlorid (132 mmol) als wäßrige Lösung vorgelegt (Katalysatorkonzentration in der wäßrigen Phase: 39,0 Gew.-%), mit der aus Tabelle 4 ersichtlichen Menge des jeweiligen Alkohols versetzt und auf die angegebene Temperatur aufgeheizt. Ansschließend wurde unter Rühren (900 Upm) und Sauerstoffbegasung über eine Fritte innerhalb von 3 h eine Lösung aus 12 g 2,3,6-Trimethylphenol (88 mmol) in der aus Tabelle 4 abzulesenden Menge des jeweiligen Alkohols zugetropft. Nach Beendigung der Zugabe wurde noch die aus Tabelle 4 ersichtliche Zeit bei der angegebenen Temperatur unter Sauerstoffbegasung nachgerührt und der Reaktionsverlauf per HPLC verfolgt. Nach Ablauf der Reaktion wurden die Phasen getrennt, die organische Phase 2mal mit Wasser gewaschen und die TMQ-Ausbeute durch HPLC mit externem Standard ermittelt.

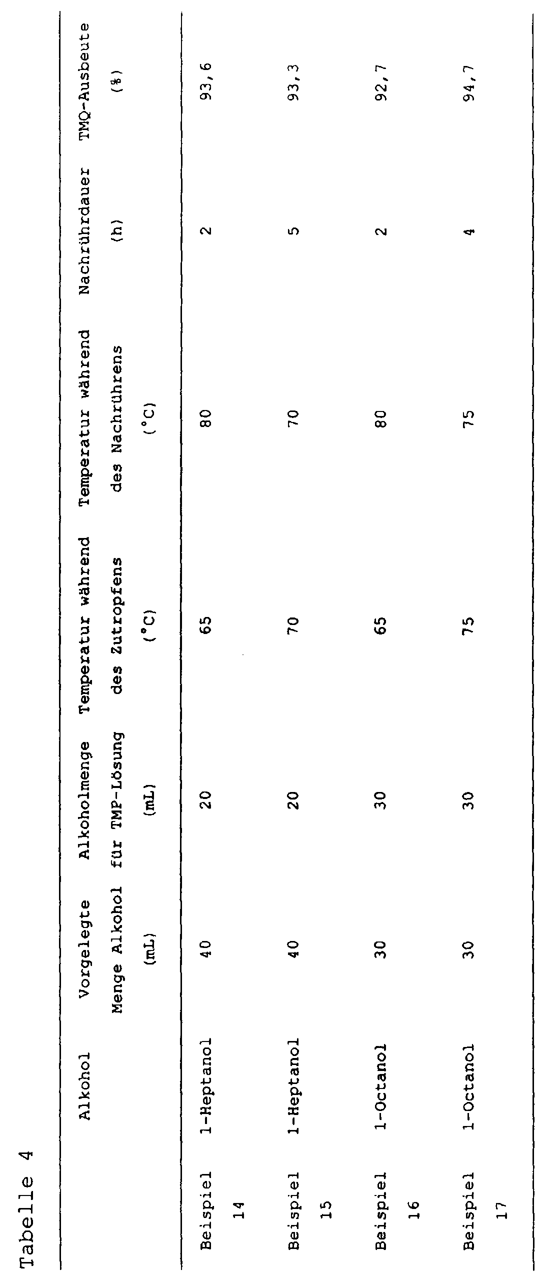

## Patentansprüche

1. Verfahren zur Herstellung von 2,3,5-Trimethyl-p-benzochinon durch Oxidation von Trimethylphenol mit Sauerstoff oder einem Sauerstoff enthaltenden Gasgemisch in Gegenwart eines mindestens Kupferhalogenid enthaltenden Katalysators in einem zweiphasigen Reaktionsmedium bei erhöhter Temperatur, dadurch gekennzeichnet, daß man die Umsetzung in einem Reaktionsmedium bestehend aus Wasser und einem aliphatischen Alkohol mit 5 bis 10 C-Atomen oder aus Wasser und einem aliphatischen Alkohol mit 1 bis 4 C-Atomen und einem aromatischen Kohlenwasserstoff in Gegenwart eines aus einem Kupferhalogenid und zusätzlich einem Übergangsmetallhalogenid der Gruppe Eisen, Chrom, Mangan, Kobalt, Nickel, Zink oder einem Halogenid eines Elementes der seltenen Erden bestehenden Katalysatorsystems bei Temperaturen zwischen 20 und 120°C durchführt.

2. Verfahren zur Herstellung von 2,3,5-Trimethyl-p-benzochinon gemaß Anspruch 1,
dadurch gekennzeichnet,
daß man die Umsetzung in einem Reaktionsmedium bestehend aus Wasser und einem Gemisch aus einem aliphatischen Alkohol mit 1 bis 4 C-Atomen und Toluol oder Benzol durchführt.

3. Verfahren zur Herstellung von 2,3,5-Trimethyl-p-benzochinon gemäß Anspruch 1,
dadurch gekennzeichnet,
daß man die Umsetzung in einem Reaktionsmedium bestehend aus Wasser und 1-Hexanol, 1-Heptanol, 2-Ethylhexanol oder 1-Octanol durchführt.

4. Verfahren zur Herstellung von 2,3,5-Trimethyl-p-benzochinon gemäß Anspruch 1,
dadurch gekennzeichnet,
daß man als Übergangsmetallhalogenid Chrom (III), Mangan (II) oder Kobalt(II)chlorid verwendet.

5. Verfahren zur Herstellung von 2,3,5-Trimethyl-p-benzochinon gemäß Anspruch 1,
dadurch gekennzeichnet,
daß man als Halogenid eines Elementes der seltenen Erden Cer(III)chlorid verwendet.
